Europäisches Patentamt

European Patent Office

Office européen des brevets

(19)

(11) Numéro de publication: **0 252 846 B1**

(12)

# FASCICULE DE BREVET EUROPEEN

(45) Date de publication de fascicule du brevet:
26.06.91

(51) Int. Cl.⁵: **B01J 23/80**, B01J 37/34,
C07C 29/15

(21) Numéro de dépôt: 87401609.0

(22) Date de dépôt: 08.07.87

(54) **Catalyseur de synthèse du méthanol et son application à la synthèse du méthanol.**

(30) Priorité: **08.07.86 FR 8609908**

(43) Date de publication de la demande:
**13.01.88 Bulletin 88/02**

(45) Mention de la délivrance du brevet:
**26.06.91 Bulletin 91/26**

(84) Etats contractants désignés:
**AT BE DE ES GB GR IT LU NL SE**

(56) Documents cités:
**EP-A- 0 079 565**
**EP-A- 0 152 809**
**FR-A- 2 564 748**

(73) Titulaire: **SOLLAC**
**Immeuble Elysées, La Défense 29 Le Parvis**
**F-92800 PUTEAUX(FR)**

(72) Inventeur: **Cordier, Jean Armand**
**La Rougeville 48, rue Henri Barbusse**
**F-59880 St Saulve(FR)**
Inventeur: **Petit, Francis Paul**
**13 Allée de la Clairière**
**F-59650 Villeneuve D'Asq(FR)**

(74) Mandataire: **Polus, Camille et al**
**c/o Cabinet Lavoix 2, Place d'Estienne d'Or-**
**ves**
**F-75441 Paris Cedex 09(FR)**

Rank Xerox (UK) Business Services

EP 0 252 846 B1

## Description

La présente invention est relative a un catalyseur de synthèse du méthanol, son procédé de préparation et son application a la synthèse du méthanol par décomposition du formiate de méthyle ou par hydrocondensation du monoxyde de carbone ou de l'anhydride carbonique.

On connait d'après le brevet FR-A-2 564 748 un catalyseur d'hydrocondensation du monoxyde de carbone obtenu par mise en contact de :

a) un complexe de formule M(L)n dans laquelle

M est un métal choisi parmi le fer, le ruthénium, le nickel, le rhodium et le molybdène, n est le nombre de ligands complexant M et

L est un ligand ionique ou covalent

b) un activateur à base d'un complexe métallique de formule M' Rm dans lequel M' est notamment le zinc et R un radical hydrocarboné

c) un élément choisi parmi divers composés tels que des amines ou des diènes conjugués.

Une application de ce catalyseur est décrite pour la fabrication d'un mélange d'hydrocarbures et de composés oxygénés aliphatiques tels qu'un mélange prépondérant d'hydrocarbures aliphatiques en $C_1$-$C_3$ et d'alcools aliphatiques en $C_1$-$C_2$ .

Ce catalyseur manque cependant de sélectivité et nécessite une étape ultérieure de séparation des différentes espèces qui est délicate et coûteuse.

On connait, en outre, d'après EP-A-0 152 809 un précurseur de catalyseur destiné à la synthèse du méthanol et d'alcools supérieurs contenant de l'oxyde de cuivre, de zinc, d'aluminium et au moins un carbonate qu oxyde de métal alcalin. Lors de la synthèse, il se produit une réduction d'au moins une partie du cuivre en une entité catalytiquement active.

L'invention a pour but de fournir un catalyseur sélectif de synthèse du méthanol ayant un excellent rendement.

Elle a ainsi pour objet un catalyseur de synthèse du méthanol, caractérisé en ce qu'il contient du cuivre (I) et du zinc (II) et est obtenu par un procédé d'oxydo réduction chimique ou électrochimique.

Selon un premier mode de réalisation le catalyseur est obtenu par voie. chimique en mettant en contact :

a) un complexe de cuivre (II) de formule Cu(L)n dans laquelle L est un ligand ionique ou covalent, et n est le nombre de ligands complexant Cu

b) un composé organique de zinc de formule Zn $(R)_2$ dans laquelle R est un groupe hydrocarboné

dans un solvant organique inerte vis à vis des réactifs.

Le ligand L peut être notamment choisi parmi les cétones, (en particulier l'acétylacétone) et les anions carboxylates (en particulier acétate), chlorates et halogénes.

Le groupe hydrocarboné R peut être notamment un groupe alkyle en $C_1$-$C_{12}$ mais peut être également un groupe cycloalkyle, aryle, aralkyle ou alkylaryle. Comme exemples de composé $Zn(R)_2$ on peut citer les (dialkyl $C_1$-$C_{12}$) zinc et notamment le diéthylzinc.

Le rapport molaire composé de zinc/complexe de cuivre est avantageusement de 0.5 à 5,0.

Le solvant peut être tout solvant organique inerte vis à vis des réactifs, par exemple des hydrocarbures saturés, des hydrocarbures aromatiques, des solvants chlorés. Le formiate de méthyle qui constitue l'un des réactifs possible pour la synthèse du méthanol constitue également un solvant particulièrement approprié.

Selon un second mode de réalisation, le catalyseur est obtenu par voie électrochimique.

Dans une première variante, on soumet à une électrolyse à potentiel cathodique imposé un complexe de cuivre (II) de formule Cu(L)n tel que défini précédemment dans une solution de formiate de méthyle, chargée en sel de fond, en présence d'un système à 3 électrodes comportant une anode soluble en zinc et une électrode de référence.

Dans une seconde variante, on utilise un complexe de zinc de formule Zn(L)n, dans laquelle L est tel que défini précédemment et n est le nombre de ligands complexant Zn dans une solution de formiate de méthyle, chargée en sel de fond, en présence d'un système à trois électrodes comportant une anode soluble en cuivre et une électrode de référence.

Le sel de fond est généralement un sel de tétra alkyl ammonium, particulièrement l'hexafluorophosphate de tétrabutylammonium.

L'électrode de référence peut être notamment une électrode d'argent Ag/AgCl/Cl⁻ .

Le potentiel cathodique imposé est de préférence d'environ -1600 mV par rapport à une électrode de référence Ag/AgCl/Cl⁻ = $2.10^{-2}$M, dans le cas de l' anode soluble en Zn.

Le potentiel cathodique imposé est de préférence d'environ - 1600 mV par rapport à une électrode de

2

référence Ag/AgCl/Cl⁻ = $2.10^{-2}$ M dans le cas de l'anode soluble en Cu.

On obtient une suspension que l'on peut utiliser telle quelle ou après filtration.

Les catalyseurs selon la présente invention peuvent être utilisés pour la préparation du méthanol avec un excellent rendement et une grande sélectivité.

Selon un premier mode de réalisation de la synthèse du méthanol, on effectue une décarbonylation du formiate de méthyle en présence d'un catalyseur selon l'invention, par chauffage à une température supérieure a $180^\circ$ C, sous une atmosphère résiduaire d'oxyde de carbone de 2 à $20.10^5$Pa.

Selon un second mode de réalisation, de préparation du méthanol, on effectue une hydrocondensation de l'oxyde de carbone ou de l'anhydride carboniques sous une pression d' hydrogéne de 2 à $80 .10^5$Pa, par chauffage à une température de 150 à $250^\circ$ C en présence du catalyseur en suspension dans un solvant liquide dans les conditions de la réaction.

Le solvant peut être notamment du Santotherm 66, un solvant constitué d'un mélange de terphényles hydrogénés, commercialisé par la Société MONSANTO.

Dans la réaction d' hydrocondensation le rapport molaire $CO/H_2$ ou $CO_2/H_2$ peut être compris entre 0,1 et 2.

Les exemples 1 à 3 ci-après illustrent la préparation d'un catalyseur selon l'invention. Les exemples 4 à 7 illustrent la synthèse du méthanol à l'aide des catalyseurs selon l'invention.

Exemple 1 : Catalyseur obtenu par voie chimique selon le premier mode de réalisation.

Dans un tube de Schlenk, on introduit 1g d'acétylacétonate de cuivre ( cuivre II ) ( 3,8 m Moles) préalablement séché sous vide que l'on dissout dans 30 ml de formiate de méthyle. La solution bleutée obtenue est aussitôt réduite sous azote par 0,4 ml de diéthylzinc. La solution vire au vert sale puis au noir avec apparition d'une suspension.

Les degrés d'oxydation de cuivre et du zinc dans le catalyseur ont été déterminés par spectrométrie de photo électrons induits par rayons X.

Les données ont été obtenues avec la radiation K $\propto$ 1/2 Al (hV = 1486,6) et une précision sur la position des photopics de ± 0,2 eV. Les énergies de photoémission et des pics Auger ont été déterminées en assignant au pic 1S du carbone de surface une énergie de 285 eV.

Les degrés d'oxydation du cuivre et du zinc ont été déterminés en comparant les énergies expérimentales correspondant au pic Auger du cuivre et aux bandes $ZnL3$ M $_{45}$ et $Zn_{2p}$ 3/2, aux énergies de ces bandes pour des composés connus du cuivre (I) et du zinc (II).

Cette détermination a confirmé que le catalyseur contient Cu (I) et Zn (II).

Exemple 2 : Catalyseur obtenu par voie électrochimique selon le second mode de réalisation, première variante.

Les études voltampérométriques effectuées sur l'acétylacétonate de cuivre dans le formiate de méthyle en présence d'un sel de fond à base de tétrabutylammonium révélent la présence de 2 paliers de réduction respectivement situés à 800 et 1600 mV.

Compte tenu de ce résultat, l'électrolyse à potentiel cathodique imposé (Vc = -1600 mv/Ag⁺/AgCl/ Cl⁻ = 0,02 M) dans le formiate de méthlyle est effectuée avec une anode soluble de zinc sur une solution de 30 ml de formiate de méthyle dans laquelle sont dissous 1g d'acétylacétonate de cuivre et 500 mg de $NBu_4$ $PF_6$ . Après le passage d'environ deux équivalents d'électron, on obtient une suspension noir que l'on peut éventuellement filtrer (récupération d'une poudre noir utilisable en synthèse catalytique du méthanol) ou utiliser directement pour la décarbonylation du formiate de méthyle.

L'analyse des degrés d'oxydation effectuée comme décrit à l' exemple 1 a confirmé l' existence du cuivre a l'état monovalent et du zinc à l'état divalent dans le catalyseur.

L'analyse par microsonde électronique de Castaing de ce catalyseur a donné les compositions atomique et massique suivantes :
- atomique : Cu : 60, 15% Zn : 37,37% soit un rapport Cu/ Zn = 1,6
- masse : Cu : 38 ,01% Zn : 24 ,08% (C, O, H non, comptabilisées)

Exemple 3 : Catalyseur obtenu par voie électrochimique selon le second mode de réalisation, seconde variante:

1,11 g d'acétylacétonate de zinc en présence d'un sel de fond (500 mg $NBu_4PF_6$ ). dissous dans 30 ml de formiate de méthyle , sont électrolysés à potentiel cathodique imposé de - 1, 570 mV/Ag/AgCl/Cl⁻ = $2.10^{-2}$ M dans le formiate de méthyle en utilisant une anode soluble de cuivre. Après passage d'environ 2 équivalents d'électron , on obtient une suspension noire qui peut être utilisée telle quelle pour la décarbonylation du formiate de méthyle.

Qu'il s'agisse du matériau solide récupéré ou de la suspension, le produit d'oxydo-réduction à base de Cu (I) et Zn (II) préparé à partir de l'un des exemples 1 à 3, possède les mêmes propriétés catalytiques qui sont mises en évidence dans les exemples ci-après.

Exemple 4 :Préparation du méthanol par décarbonylation du formiate de méthyle.

La suspension préalablement préparée à l'exemple 1 est transférée sous léger balayage de CO dans un autoclave. Le réacteur est alors pressurisé à la température ambiante sous une pression de 1 à 2 $10^6$ Pa de CO.

L'ensemble est ensuite chauffé très rapidement à 200° C et mis sous agitation. On constate alors une augmentation progressive et importante de la pression, qui se stabilise au bout de 15 heures environ.

L'agitation est alors arrêtée et le réacteur rapidement refroidi.

Des analyses effectuées sur la phase gazeuse révèlent que celle-ci est composée de monoxyde de carbone pratiquement pur (99%) et de légères traces de $CO_2$. La phase liquide ne contient que du méthanol pur et du formiate de méthyle non converti (30%).

Exemple 5 : Préparation du méthanol par décarbonylation du formiate de méthyle.

La suspension obtenue à l' exemple 3 est transférée sous balayage de CO dans un réacteur à la température ambiante sous une pression de $1,5.10^6$ Pa de CO.

L'ensemble est ensuite porté à 200° C et mis sous agitation . On constate une augmentation de la pression qui se stabilise au bout de 18 heures.

Des analyses effectuées sous la phase gazeuse révèlent que celle- ci est composée de monoxyde de carbone pur ( 98%) et de $CO_2$ (2%). La phase liquide ne contient que du méthanol pur et du formiate de méthyle non converti (20%).

Exemple 6 : Préparation du méthanol par hydrocondensation du CO.

Dans un autoclave de 100 ml, on introduit 30 g de Santotherm 66 (fluide caloporteur commercialisé par la société Monsanto constitué d'un mélange de tephényles hydrogénés qui possède une fourchette d' ébullition allant de 340 à 400° C) et 10 m Moles du matériau catalytique précédemment décrit à l'exemple 2.

L'ensemble est mis sous pression de CO-H2 (1/1) ($20 \leqslant P \leqslant 80.10^5$ Pa, agité et chauffé ($165 < T \leqslant 225$° C). Au bout d'une heure, on arrête la réaction et on procède au dosage des produits formés.

L'ensemble des résultats obtenus est donné dans les tableaux 1 et 2.

Tableau 1 : Préparation du méthanol - Influence de la température

| T (°C) | 165 | 181 | 199 | 222 | 225 |
|--------|-----|-----|-----|-----|-----|
| TTU (%) | 0,31 | 0,38 | 1,26 | 1,65 | 3,41 |
| VR ($h^{-1}$) | 0,03 | 0,03 | 0,11 | 0,12 | 0,26 |
| CH3 OH(%) | 95 | 99 | 98,6 | 99,1 | 98,9 |

P = 50 .$10^5$Pa CO/H2 = 1 Bilan statique : 1 h TTU : taux de transformation utile ( masse de carbone récupérée sous forme de produits/masse de carbone introduite sous forme de CO) × 100.

VR = vitesse de rotation = nombre de moles de CO consommées/nombre de moles de catalyseur × unité de temps).

Tableau 2 : Préparation du méthanol - Influence de la pression

| P ($10^5$Pa) | 20 | 35 | 50 | 60 | 80 |
|---|---|---|---|---|---|
| TTU (%) | 0,5 | 0,58 | 1,26 | 1,28 | 1,90 |
| VR ($h^{-1}$) | 0,01 | 0,03 | 0,11 | 0,12 | 0,20 |
| Méthanol (%) | 75,4 | 94,9 | 98,6 | 99,2 | 99,5 |

T = 200°C    CO/H2 = 1    t = 1h

Des résultats analogues peuvent être obtenus avec le catalyseur obtenu à l'exemple 1.

Exemple 7 : Préparation du méthanol par hydrocondensation du CO2.

Le protocole est identique à celui de l'exemple 6, mais la pressurisation est effectuée avec un mélange CO2/H2 (1/3) entre 20 et 80 .$10^5$Pa. Les résultats sont donnés dans les tableaux 3 et 4.

**Tableau 3 : Réduction de l'anhydride carbonique - Influence de la pression**

| $P\ (10^5\ Pa)$ | 20 | 30 | 50 | 60 | 80 |
|---|---|---|---|---|---|
| TTU (%) | 2 | 1,8 | 1,8 | 1,8 | 1,85 |
| $VR\ (h^{-1})$ | 0,02 | 0,04 | 0,06 | 0,09 | 0,12 |
| Méthanol (%) | 97,5 | 98,9 | 98,2 | 98,9 | 99,5 |

$T = 200°C$    $CO2/H2 = 1/3$    Bilan statique : 1 h

**Tableau 4 : Réduction de l'anhydride carbonique - Influence du temps de contact**

| $V\ (h^{-1})$ | 520 | 270 | 225 | 107 | 35 |
|---|---|---|---|---|---|
| TTU (%) | 1,1 | 0,6 | 0,6 | 0,9 | 0,7 |
| $VR\ (h^{-1})$ | 0,2 | 0,06 | 0,04 | 0,03 | 0,01 |
| CH3OH (%) | 99,3 | 99 | 98,7 | 98,4 | 95 |

$P = 50\ 10^5 Pa$   $T = 200°C$   $CO2/H2 = 1/3$ bilan dynamique : 1 h.

**Revendications**

1. Catalyseur de synthèse du méthanol, caractérisé en ce qu'il contient du cuivre à l'état monovalent, Cu (I) et du zinc à l'état divalent,(II) et est obtenu par un procédé d'oxydoréduction chimique ou électrochimique.

2. Catalyseur selon la revendication 1, caractérisé en ce qu'il est obtenu par voie chimique en mettant en contact :

a) un complexe de cuivre (II) de formule Cu(L)n dans laquelle L est un ligand ionique ou covalent, et n est le nombre de ligands complexant Cu

b) un composé organique de zinc de formule Zn (R)$_2$ dans laquelle R est un groupe hydrocarboné dans un solvant organique inerte vis à vis des complexes.

3. Catalyseur selon la revendication 2, caractérisé en ce que le rapport molaire composé de zinc/complexe de cuivre est de 0,2 à 5,0.

4. Catalyseur selon la revendication 1, caractérisé en ce qu'il est obtenu en soumettant à une électrolyse à potentiel cathodique imposé un complexe de cuivre (II) de formule Cu (L)n, dans lequelle L est un ligand ionique ou covalent et n est le nombre de ligands complexant Cu, dans une solution de formiate de méthyle, chargée en sel de fond en présence d'un système à 3 électrodes comportant une anode soluble en zinc et une électrode de référence.

5. Catalyseur selon la revendication 1, caractérisé en ce qu'il est obtenu en soumettant à une électrolyse à potentiel cathodique imposé un sel de Zn de formule Zn(L)n, dans lequelle L est un ligand ionique ou covalent et n est le nombre de ligands complexant Zn, dans une solution de formiate de méthyle, chargée en sel de fond en présence d'un système à trois électrodes comportant une anode soluble en cuivre et une électrode de référence.

6. Catalyseur selon la revendication 4 ou 5, caractérisé en ce que le sel de fond est le sel de tétraalkylammonium, notamment l'hexafluorophosphate de tétrabutylammonium.

7. Catalyseur selon la revendication 4, caractérisé en ce que le potentiel imposé est d'environ - 1,6 V/par rapport à une électrode de référence Ag/AgCl/Cl$^-$ = 2.10$^{-2}$ M dans le cas de l'anode soluble de Zn.

8. Catalyseur selon la revendication 5, caractérisé en ce que le potentiel imposé est d'environ - 1,6 V/par rapport à une électrode de référence Ag/AgCl/Cl$^-$ = 2.10$^{-2}$ M dans le cas de l'anode soluble en Cu.

9. Procédé de préparation du méthanol par décarbonylation du formiate de méthyle, caractérisé en ce que on chauffe le formiate de méthtyle à une température supérieure à 180 $^\circ$C et sous une atmosphère résiduaire d'oxyde de carbone de 2 à 20.10$^5$ Pa, en présence du catalyseur selon l'une quelconque des revendications 1 à 8.

10. Procédé de préparation du méthanol par hydrocondensation de l'oxyde de carbone ou de l'anhydride carbonique, caractérisé en ce qu'on effectue l'hydrocondensation à une température de 150 à 250 $^\circ$C et sous une pression d hydrogéne de 2 à 80.10$^5$ Pa en présence d' un catalyseur selon l'une quelconque des revendications 1 à 8 en suspension dans un solvant liquide dans les conditions de la réaction.

**Claims**

1. Catalyst for the synthesis of methanol, characterised in that it contains copper in the monovalent state Cu (I) and zinc in the divalent state Zn (II) and is obtained by a process of chemical or electrochemical oxidation-reduction.

2. Catalyst according to claim 1, characterised in that it is obtained by a chemical method by contacting:
   a) a copper(II) complex of the formula Cu(L)n, wherein L is an ionic or covalent ligand, and n is the number of Gin complexing ligands,
   b) an organic zinc compound of formula Zn (R)$_2$ wherein R is a hydrocarbon group in an organic solvent which is inert with respect to complexes.

3. Catalyst according to claim 2, characterised in that the molar ratio of the zinc compound to the copper complex is from 0.2 to 5.0.

4. Catalyst according to claim 1, characterised in that it is obtained by subjecting a copper(II) complex of formula Gin (L)n, wherein L is an ionic or covalent ligand and n is the number of Cu complexing ligands, is subjected to electrolysis with an imposed cathodic potential, in a solution of methyl formate,

charged with base salt in the presence of a 3-electrode system comprising a soluble zinc anode and a reference electrode.

5. Catalyst according to claim 1, characterised in that it is obtained by subjecting a Zn salt of formula Zn-(L)n, wherein L is an ionic or covalent ligand and n is the number of Zn complexing ligands, to electrolysis with an imposed cathodic potential, in a solution of methyl formate charged with base salt in the presence of a three-electrode system comprising a soluble copper anode and a reference electrode.

6. Catalyst according to claim 4 or 5, characterised in that the base salt is tetraalkylammonium salt, notably tetrabutylammonium hexafluorophosphate.

7. Catalyst according to claim 4, characterised in that the imposed potential is about -1.6 V in relation to a reference electrode Ag/AgCl/Cl⁻ = 2.10⁻² M in the case of the soluble Zn anode.

8. Catalyst according to claim 5, characterised in that the imposed potential is approximately -1.6 V in relation to a reference electrode Ag/AgCl/Cl⁻¹ = 2.10⁻² M in the case of the soluble Cu anode.

9. Process for preparing methanol by decarbonylation of methyl formate, characterised in that the methyl formate is heated to a temperature above 180°C under a residual carbon monoxide atmosphere of 2 to 20.10⁵ Pa, in the presence of the catalyst according to any one of claims 1 to 8.

10. Process for preparing methanol by hydrocondensation of carbon monoxide or carbonic anhydride, characterised in that the hydrocondensation is carried out at a temperature from 150 to 250°C and under a hydrogen pressure of 2 to 80.10⁵ Pa in the presence of a catalyst according to any one of claims 1 to 8 suspended in a liquid solvent under the reaction conditions.

**Ansprüche**

1. Katalysator für die Methanolsynthese, dadurch charakterisiert, daß er Kupfer als monovalentes Cu (I) und, Zink als divalentes Zn (II) enthält und durch ein chemisches oder elektrochemisches Rodoxverfahren erhalten wird.

2. Katalysator nach Anspruch 1, charakterisiert dadurch, daß er chemisch hergestellt wird durch Kontaktieren:
a) eines Kupfer (2) komplexes der Formel Cu(L)n, wobei L ein ionischer oder kovalenter Ligand und n die Zahl der das Kupfer komplexierenden Liganden sind, mit
b) einer organischen Zinkverbindung der Formel Zn(R)₂, in der R eine Kohlenwasserstoffgruppe ist, in einem gegenüber den Komplexen inerten organischen Lösungsmittel.

3. Katalysator nach Anspruch 2, dadurch gekennzeichnet, daß das molare Verhältnis Zinkverbindung/Kupferkomplex 0,2 bis 5,0 beträgt.

4. Katalysator nach Anspruch 1, dadurch gekennzeichnet, daß er erhalten wird durch Elektrolyse mit Kathodenpotential eines Kupfer(II)komplexes der Formel Cu(L)n, in dar L ein ionischer oder kovalenter Ligand und n die Zahl der Kupfer komplexierenden Liganden sind, in einer Losung von Methylformiat, die ein Grundsalz enthält, in Gegenwart eines Systems aus drei Elektroden, wobei eine Zink-Lösungsanode und eine Referenzelektrode vorhanden sind.

5. Katalysator nach Anspruch 1, dadurch gekennzeichnet, daß er erhalten wird durch Elektrolyse mit Kathodenpotential eines Zinksalzes der Formel Zn(L)n, wobei L ein ionischer oder kovalenter Ligand und n die Zahl der Zink komplexierenden Liganden sind, in einer Methylformiatlösung mit Gehalt an Grundsalz in Gegenwart eines Systems von drei Elektroden, wobei eine KupferLösungsanode und eine Referenzelektrode vorhanden sind.

6. Katalysator nach Anspruch 4 oder 5, dadurch gekennzeichnet, daß das Grundsalz ein Tetraalkylamoniumsalz, insbesondere das Hexafluorphosphat von Tetrabutylamonium ist.

7.  Katalysator nach Anspruch 4, dadurch gekennzeichnet, daß das angelegte Potential etwa -1,6 V/bezogen auf eine Bezugselektrode Ag/AgCl/Cl⁻ = 2.10⁻² M im Fall der Zn-Lösungsanode beträgt.

8.  Katalysator nach Anspruch 5, dadurch gekennzeichnet, daß dar angelegte Potential etwa -1,6 V/bezogen auf eine Referenzelektrode Ag/AgCl/Cl⁻ = 2.10⁻² H im Fall der Cu-Lösungsanode beträgt.

9.  Verfahren zur Herstellung von Methanol durch Decarbonylierung von Methylformiat, dadurch gekennzeichnet, daß man das Methylformiat auf eine Temperatur oberhalb 180° C und unter einer verbleibenden Atmosphäre von Kohlenmonoxid von 2 bis 20 x 10⁵ Pa, in Gegenwart des Katalysators nach einem der Ansprüche 1 bis 8 erhitzt.

10. Verfahren zur Herstellung von Methanol durch Hydrokondensation von Kohlenmonoxid oder Kohlendioxid, dadurch gekennzeichnet, daß man die Hydrokondensation bei einer Temperatur von 150 bis 250° C unter einem Wasserstoffdruck von 2 bis 80 x 10⁵ Pa in Gegenwart eines Katalysators gemäß einem der Ansprüche 1 bis 8 in Suspension in einem Lösungsmittel, das unter den Reaktionsbedingungen flüssig ist, durchführt,